(19) Europäisches Patentamt
European Patent Office
Office européen des brevets

(11) **EP 3 925 568 A1**

(12) **EUROPEAN PATENT APPLICATION**
published in accordance with Art. 153(4) EPC

(43) Date of publication:
**22.12.2021 Bulletin 2021/51**

(21) Application number: **20745910.8**

(22) Date of filing: **22.01.2020**

(51) Int Cl.:
*A61C 8/00* (2006.01)     *A61B 34/10* (2016.01)
*A61C 19/04* (2006.01)

(86) International application number:
**PCT/JP2020/002160**

(87) International publication number:
**WO 2020/153410 (30.07.2020 Gazette 2020/31)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **22.01.2019 JP 2019008579
22.01.2019 JP 2019008578**

(71) Applicant: **Safe Approach Medical Inc.
Fukuoka-shi, Fukuoka 814-0001 (JP)**

(72) Inventors:
• **CHO Byunghyun
Fukuoka-shi, Fukuoka 812-0054 (JP)**
• **OUCHIDA Riichi
Fukuoka-shi, Fukuoka 812-0054 (JP)**

(74) Representative: **Plougmann Vingtoft a/s
Strandvejen 70
2900 Hellerup (DK)**

(54) **SURGICAL PROCEDURE ASSISTANCE SYSTEM, TREATMENT DEVICE, AND PLATE**

(57) This plate 10 used by being fixed to a stent 15 mounted on the dentition of a patient is provided with: a plurality of CT markers 11; an attachment/detachment unit 12 for connecting a reference frame 20 that is provided with a plurality of infrared markers 25 in which the relative positional relationship between the respective CT markers and the infrared markers 25 are known when the attachment/detachment unit 12 is connected to the plate 10; and a recessed part 16 used for a measurement of accuracy of positioning between the position coordinates of the plurality of CT markers 11 on a three-dimensional reconstructed image of the CT including the plate 10 in a subject and the position coordinates calculated on the basis of the position coordinates of the plurality of infrared markers 25 which are the position coordinates of the CT markers 11 in the actual space in which the plate 10 is present.

FIG. 2

# Description

## TECHNICAL FIELD

The present disclosure relates to a surgical procedure support system for a dental implant, and to a processing apparatus and a plate that are used in the surgical procedure support system.

## BACKGROUND OF THE INVENTION

Conventionally, in dental implant treatments, a position of an implant is visually confirmed and a hole is drilled in the jawbone using a dental drill. Also, a technique of accurately guiding a position of an implant using a plaster model and a surgical guide, and designing an implant and performing dental implant treatments while looking at an image, in which a condition of the oral cavity is three-dimensionally represented, have been practiced.

Patent Document 1 discloses a surgical procedure support system for creating design information by perceiving, intuitively and in real time, an appropriate position to embed an implant, and the system includes: a CT imaging apparatus that captures a CT image together with a reference marker for indicating a reference position of the teeth; a first position marker, the position of which relative to the reference marker is fixed; a second position marker that specifies a position and a direction of an operation part; a three-dimensional model storage part that stores three-dimensional-model information on the basis of the CT image and three-dimensional-model information concerning a virtual implant to be mounted in a direction perpendicular to a longitudinal direction of the operation part; a display control part that displays the three-dimensional-model information in accordance with movement of the operation part and a patient by performing positional alignment on the basis of position information concerning three markers, and a capture part that stores, as design information, a desired position and angle when the three-dimensional model of the virtual implant is displayed at the desired position and angle.

Patent Document 2 discloses a system for aligning (registration of) a human jaw using a CT image in a dental navigation system.

## PRIOR ART

## PATENT DOCUMENTS

Patent Document 1: WO-A-2018/088146
Patent Document 2: U.S. Patent Application Publication No. 2018/0279975

## SUMMARY OF INVENTION

## PROBLEMS TO BE SOLVED BY THE INVENTION

In the techniques described in Patent Documents 1 and 2, in the surgical procedure support system for a dental implant, a dentist in a dental clinic has to process CT image data and generate a three-dimensional reconstructed image, which is a three-dimensional model related to the CT image data. Since these processes are labor-intensive and time-consuming tasks, it is required to reduce the work involved in these processes in the dental clinic. For example, when a part of data processing is commissioned to another party between personnel using different systems, formats may differ or a fatal error may occur, which does not necessarily lead to improvement in work efficiency.

The present disclosure focuses on this point, and its object is to provide a technique for reducing the workload of a practitioner engaged in dental implant treatments.

## MEANS FOR SOLVING THE PROBLEMS

A first aspect of the present disclosure is a plate to be used by being fixed to a stent to be mounted on a row of teeth of a patient. The place includes a plurality of computed tomography (CT) markers; a detachable part that is configured to connect a reference frame provided with a plurality of infrared markers whose relative positional relationship with the respective CT markers when the reference frame is connected to the plate is known, and a recessed part that is configured to be used in measuring registration accuracy between (i) position coordinates of the plurality of CT markers in a three-dimensional reconstructed CT image including the plate as an object and (ii) position coordinates of the CT markers in real space in which the plate exists, the position coordinates being calculated on the basis of position coordinates of the plurality of infrared markers.

A second aspect of the present disclosure is a processing apparatus. This apparatus includes a storage part that stores digital imaging and communications in medicine (DICOM) data relating to a CT image captured while a stent, to which the plate described above is fixed, is attached to the teeth of a patient, the DICOM data being acquired via a network; a three-dimensional model generation part that generates a three-dimensional reconstructed CT image on the basis of the DICOM data; a position acquisition part that receives a designation of positions of the plurality of CT markers in the three-dimensional reconstructed image from a user of the processing apparatus; a three-dimensional measurement part that measures the position coordinates of the plurality of infrared markers in a state where the stent, to which the plate connected with the reference frame is fixed, is mounted on a tooth-form plaster model of the patient; a coordinate calculation part that calculates position coordinates of the plurality of CT markers provided on the plate on the basis of a relative positional relationship between the position coordinates of the plurality of infrared markers and the plurality of CT markers; and a registration part that implements a registration of the three-dimensional reconstructed image and the tooth-form plaster model on the basis of the positions of the plurality of CT markers designated by the user and the position coordinates calculated by the coordinate calculation part.

The reference frame may rotate at a plurality of predetermined angles with respect to the plate fixed to the stent mounted on the tooth-form plaster model, and the registration part may generate a transformation matrix for transforming the positions of the plurality of infrared markers provided in the reference frame into the positions of the plurality of CT markers in the three-dimensional reconstructed image for each of the predetermined angles, on the basis of (i) known design information regarding the relative positional relationship between the plurality of infrared markers provided in the reference frame and the plurality of CT markers provided in the plate and (ii) the positions of the plurality of CT markers in the three-dimensional reconstructed image.

The processing apparatus may further includes a transmission part that transmits the three-dimensional reconstructed image, the positions of the plurality of CT markers in the three-dimensional reconstructed image, and the transformation matrix for each of the predetermined angles to a transmission-source device, from which the DICOM data is transmitted, via the network.

The three-dimensional measurement part may further capture a drill frame having a plurality of drill markers whose relative positional relationship with the drill blade is known, the drill frame being detachably attached to a dental drill having a drill blade, the registration part may calculate a position of the drill blade in the three-dimensional reconstructed image on the basis of (i) position coordinates of the plurality of drill markers measured by the three-dimensional measurement part, (ii) a relative positional relationship between the plurality of drill markers and the drill blade, (iii) position coordinates of the plurality of infrared markers, and (iv) a relative positional relationship between the position coordinates of the plurality of infrared markers and the plurality of CT markers, and the processing apparatus may further include an accuracy calculation part that calculates, as the registration accuracy, an error between a position of the drill blade and a position of the recessed part in the three-dimensional reconstructed image when the user inserts the drill blade into the recessed part; and the transmission part may further transmit the registration accuracy to the transmission-source device, from which the DICOM data is transmitted, via the network.

A third aspect of the present disclosure is a surgical procedure support system for dental implant treatments including a surgical procedure support apparatus at a first facility where the dental implant treatments are performed and the processing apparatus described above at a second facility different from the first facility and connected to the surgical procedure support apparatus via a network. In this system, the surgical procedure support apparatus includes a transmission part that transmits, to the processing apparatus via the network, DICOM data relating to the CT image captured in the state where the stent, to which the plate described above is fixed, is mounted on the teeth of a patient, a reception part that receives, from the processing apparatus via the network, the three-dimensional reconstructed image, the positions of the plurality of CT markers in the three-dimensional reconstructed image, and the transformation matrix for each of the predetermined angles, and a display control part that causes a display to display the three-dimensional reconstructed image.

The surgical procedure support apparatus may further includes a position information acquisition part that measures (i) position coordinates of the plurality of infrared markers included in the reference frame and (ii) position coordinates of the plurality of drill markers included in a drill reference frame having the drill markers whose relative positional relationship with a drill blade is known, the drill reference frame being detachably attached to a dental drill having the drill blade, and a position calculation part that calculates a position of the drill blade in the three-dimensional reconstructed image on the basis of (i) the three-dimensional reconstructed image, (ii) the position coordinates of the plurality of infrared markers, (iii) the position coordinates of the plurality of drill markers, (iv) the relative positional relationship between the drill blade and the plurality of drill markers, and (v) the transformation matrix, wherein the display control part may cause the display part to display the drill blade superimposed on the three-dimensional reconstructed image.

The display control part may cause the display part to display (1) an image, which is extracted from the three-dimensional reconstructed image, including the bones into which the implant of the patient is to be embedded, (2) a predetermined implantation completion position of the implant, (3) a predicted implantation completion position of the implant calculated on the basis of the current position of the drill blade which is calculated by the position calculation part, and (4) a current position of the implant corresponding to the current position of the drill blade, in different modes.

A position information acquisition part that acquires position coordinates of a plurality of pointing markers in a pointing tool frame that has the pointing markers detachable to a pointing part for pointing out a point in real space, a relative positional relationship of the pointing markers with the pointing part is known may also be included, wherein the position calculation part may further calculate a position of the pointing part in the three-dimensional reconstructed image on the basis of (i) the three-dimensional reconstructed image, (ii) the position coordinates of the plurality of infrared markers, (iii) the position coordinates of the plurality of pointing markers, (iv) the relative positional relationship between the plurality of pointing markers and the pointing part, and (v) the transformation matrix, and the display control part may cause the display part to display a virtual plane, which is parallel to a plane whose normal is the longitudinal direction of the implant at the predetermined implantation completion position of the implant in the three-dimensional reconstructed image and a distance from the position of the pointing part in the three-dimensional reconstructed image is a predetermined distance, by superimposing the virtual plane on an image including the bones into which the implant of the patient is to be implanted and the predetermined implantation completion position of the implant.

The position information acquisition part may further acquire position coordinates of a plurality of driver markers included in a driver frame having the driver markers whose relative positional relation with a driver to be used to implant an orthodontic anchor screw is known, the driver markers being detachably attached to the driver, the position calculation part may further calculate a position of the driver in the three-dimensional reconstructed image on the basis of (i) the three-dimensional reconstructed image, (ii) the position coordinates of the plurality of infrared markers, (iii) the position coordinates of the plurality of driver markers, (iv) the relative positional relationship between the plurality of driver markers and the driver, and (v) the transformation matrix, and the display control part may cause the display part to display a virtual image in which the driver is advanced along a long axis of the driver superimposed on an image including the bones in which the orthodontic anchor screws of the patient is to be embedded.

It should be noted that any combination of the above-described constituent elements, and an aspect obtained by converting the expression of the present disclosure among methods, devices, systems, computer programs, data structures, recording media, and the like are also effective as an aspect of the present disclosure.

EFFECT OF THE INVENTION

According to the present disclosure, it is possible to provide a technique for reducing the workload of a practitioner engaged in dental implant treatments.

BRIEF DESCRIPTION OF THE DRAWINGS

FIG. 1A is a schematic diagram of a surgical procedure support system 1 for dental implant treatments, according to an embodiment.
FIG. 1B is a functional block diagram of the surgical procedure support apparatus 100 and the like.
FIG. 2A is a schematic diagram of a plate 10 and a reference frame 20, and FIG. 2B is a schematic diagram of the reference frame 20.
FIGS. 3A to 3D are each a schematic diagram of the plate 10 and the reference frame 20.
FIG. 4 shows an illustrative example of the generation of a transformation matrix.
FIG. 5 shows an example of known design information.
FIG. 6 is a schematic diagram of a manipulator 30.
FIG. 7 is a flowchart of a dental implant treatment using the surgical procedure support system 1.
FIG. 8A shows an example of a plaster model and a stent, and FIG. 8B shows an example of the plate 10.
FIGS. 9A and 9B each show an example of a CT image.
FIG. 10 shows an example of a design position in a three-dimensional reconstructed image.
FIG. 11 shows a schematic diagram of an implant-insertion-support screen.
FIG. 12 illustrates a procedure process of the implant using the surgical procedure support system 1.
FIGS. 13A to 13E each schematically show a tool for supporting a vertical-position designing of the implant.
FIGS. 14A and 14B each schematically show a tool for an orthodontic anchor screw navigation.

DETAILED DESCRIPTION OF THE INVENTION

Hereinafter, an embodiment of the present disclosure will be described with reference to the drawings.
A surgical procedure support system 1 according to the present embodiment provides support for a practitioner (in particular, a dentist) engaged in dental implant treatments, and enables various types of processing of CT image data, which conventionally have been performed in a dental clinic, to be performed in a facility different from the dental clinic (for example, a dental laboratory, etc., hereinafter referred to as a "remote support facility"), thereby reducing the workload of the practitioner.

<Outline of the surgical procedure support system>

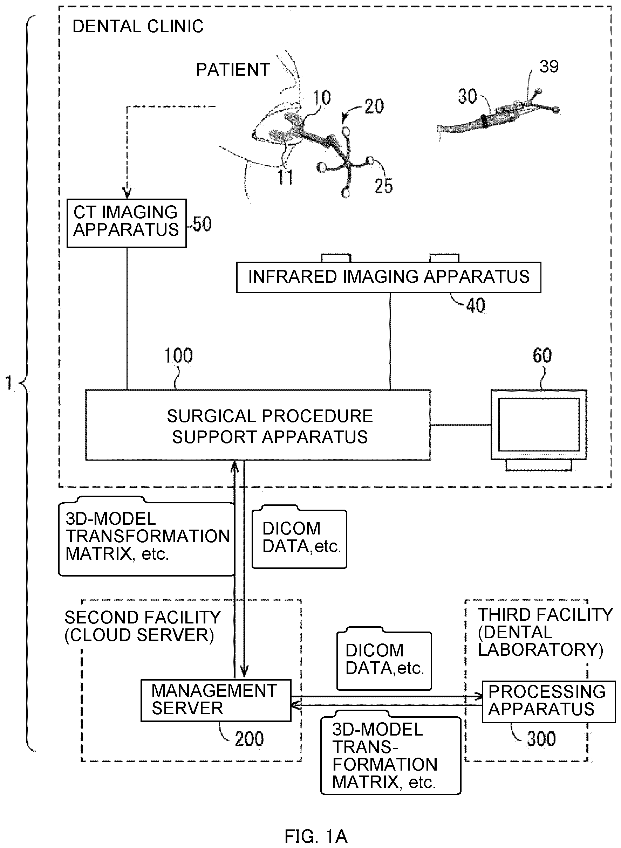

FIG. 1A is a schematic diagram of a surgical procedure support system 1 for dental implant treatments, according to the present embodiment.
The surgical procedure support system 1 includes a surgical procedure support apparatus 100 at a dental clinic (a first facility) where dental implant treatments are performed, a management server 200 (e.g., cloud server) at a second facility different from the dental clinic, and a processing apparatus 300 at one or more third facilities (remote support facilities) different from the dental clinic. Note that one of the processing apparatuses 300 may be provided in the second facility equipped with the management server 200.
The surgical procedure support apparatus 100 and the processing apparatus 300 are connected to the management server 200 through a network (e.g., the Internet) and exchange data with each other through the management server 200.

In the surgical procedure support system 1, the dental clinic is equipped with an infrared imaging apparatus 40, a CT imaging apparatus 50, a display device such as a display 60, and the surgical procedure support apparatus 100. The infrared imaging apparatus 40 captures an infrared marker 25 using infrared rays to measure position coordinates of the infrared marker 25 in real space. The CT imaging apparatus 50 captures a tomographic image of the entire or a part of the lower jaw and the upper jaw of a patient in a state where the plate 10 is placed on him/her.

Here, a CT marker 11, serving as a reference position for specifying a relationship between the plate 10 and the teeth of the patient, is embedded in the plate 10. The plate 10 is fixed to a stent which is to be mounted on a row of teeth of the patient at the time of imaging by the CT imaging apparatus 50. Therefore, the CT marker 11 can be the reference position for specifying the relationship between the plate 10 and the teeth of the patient at the time of imaging by the CT imaging apparatus 50.

In the surgical procedure support system 1 according to the embodiment, in order to specify a relative position with the CT marker 11, the plate 10 can be connected to a reference frame 20 provided with the infrared marker 25 whose relative positional relationship with the CT marker 11 is known. If the relative positional relationship between the CT marker 11 and the infrared marker 25 is fixed, position coordinates of the CT marker 11 and position coordinates of the infrared marker 25 in real space are convertible to each other by a predetermined transformation matrix. Therefore, once the position coordinates of the infrared marker 25 in real space can be specified, the position coordinates of the CT marker 11 can also be specified, and thus position coordinates in real space of the biological tissues (teeth, bones, nerves, etc.) including the teeth of the patient can be specified.

In this manner, by measuring a position of the infrared marker 25 using the three-dimensional measurement part such as the infrared imaging apparatus 40, the position coordinates of the biological tissues including the teeth of the patient can be specified by calculation. As a result, when an infrared marker 39 is attached to a manipulator 30 operated by the practitioner and the position of the infrared marker 39 is specified in relation to the infrared marker 25, the positional relationship between the manipulator 30 and the biological tissues including the teeth of the patient can be specified. An example of the manipulator 30 is a dental drill having a drill blade. A marker for a drill (a drill marker), which is the infrared marker 39, is detachable from the dental drill and provided in a reference frame for a dental drill (a drill reference frame). A relative positional relationship between a plurality of drill markers provided in the drill reference frame and the drill blade is known.

The surgical procedure support apparatus 100 acquires, from the CT imaging apparatus 50, CT image data (DICOM data) of the patient who has undergone tomography with the plate 10 fixed to his/her teeth, and stores the CT image data in a storage part 110. Since CT image data of the reference frame 20 is unnecessary when CT imaging the patient, the reference frame 20 should be removed from the plate 10.

The surgical procedure support apparatus 100 transmits DICOM data relevant to the patient to the management server 200 via the network in accordance with an instruction received from the practitioner via an input part 130. The management server 200 transmits the DICOM data received from the surgical procedure support apparatus 100 to the processing apparatus 300 in a remote support facility designated from among one or a plurality of options by the practitioner or the like.

The processing apparatus 300 generates, on the basis of the DICOM data received from the surgical procedure support apparatus 100, a three-dimensional reconstructed CT image including the plate 10 as an object. The three-dimensional reconstructed image is a three-dimensional model of the patient including the plate 10. The processing apparatus 300 segments the biological tissues of the patient included in the generated three-dimensional reconstructed image for each type of tissue. The processing apparatus 300 can perform a positional alignment (registration) of the CT marker 11 included in the plate 10 in real space and the CT marker 11 in the virtual three-dimensional reconstructed image by accepting a designation of the position of the CT marker 11 in the three-dimensional reconstructed image from an operator (e.g., dental technician) of the processing apparatus 300.

In the surgical procedure support system 1 according to the embodiment, a plaster model, which is a teeth profile design model obtained by taking a cast of a patient's teeth profile, is delivered from a dental clinic to a remote support facility in which the processing apparatus 300 is installed by mail or the like. Therefore, an operator of the processing apparatus 300 can align the CT marker 11 of the plate 10 in real space and the CT marker 11 of the three-dimensional reconstructed image by mounting a stent, to which the plate 10 connected with the reference frame 20 is fixed, on the plaster model.

Further, the plate 10 according to the embodiment includes a registration confirmation portion to be used in measuring registration accuracy between (i) the position coordinates of a plurality of CT markers 11 in the three-dimensional reconstructed image and (ii) the position coordinates of the CT markers 11 in real space in which the plate 10 exists. The registration confirmation portion is a recessed part provided on the plate 10 and is large for the drill blade of the dental drill, which is an example of the manipulator 30, to be inserted therein. When the operator of the processing apparatus 300 inserts the drill blade of the dental drill attached with the infrared marker 39 into the recessed part, the processing apparatus 300 calculates the registration accuracy of the CT markers 11 of the plate 10 in real space and the CT markers 11 in the three-dimensional reconstructed image from the positional relationship between (i) the recessed part in the three-dimensional reconstructed image and (ii) the virtual drill blade in the three-dimensional reconstructed image calculated on the basis of its relative positional relationship with the infrared marker 39.

In other words, the insertion of the drill blade into the recessed part of the plate 10 by the operator of the processing apparatus 300 in real space means that the position of the recessed part and the position of the drill blade in real space match. If the positions of the recessed part and the drill blade in the three-dimensional reconstructed image match in this state, it indicates that the registration accuracy is high. Therefore, the magnitude of the distance between the recessed part and the drill blade in the three-dimensional reconstructed image corresponds to the registration accuracy between the CT markers 11 of the plate 10 in real space and the CT markers 11 in the three-dimensional reconstructed image. The operator of the processing apparatus 300 can ensure positional accuracy of the registration by implementing the registration so that the accuracy of the registration between the CT markers 11 of the plate 10 in real space and the CT markers 11 in the three-dimensional reconstructed images becomes the positional accuracy required by the practitioner or the like.

The surgical procedure support apparatus 100 receives, via the management server 200, the registration accuracy and data relating to one or more three-dimensional reconstructed images and a transformation matrix generated after segmentation processing and registration processing in the processing apparatus 300 in the remote support facility. The practitioner or the like may designate, via the input part 130, the surgical procedure support apparatus 100 to which facility he/she wants to commission processing of DICOM data or the like in accordance with his/her preference or in consideration of congestion situations of the remote support facilities, the processing costs, and the like.

In addition, the surgical procedure support apparatus 100 displays the three-dimensional reconstructed image of the mouth (the lower jaw, the upper jaw, or both) of the patient and the plate 10 on the display 60 on the basis of data concerning the three-dimensional reconstructed image received from the management server 200. A slice image can be displayed from a three-dimensional reconstructed image.

At the time of a dental implant treatment, the patient receives treatments while the plate 10 on which the reference frame 20 is mounted is fixed to his/her teeth. At the time of treatment, the surgical procedure support apparatus 100 obtains, using a position information acquisition part 140, the relative positional relationship of the infrared markers 25 and 39 on the basis of data of images of the infrared markers 25 and 39 captured by the infrared imaging apparatus 40, and displays, on the display 60, a three-dimensional reconstructed image of the patient's mouth and the plate 10, which is based on the processed DICOM data and superimposed with a three-dimensional reconstructed image of at least the distal end portion of the manipulator 30 and treatment components such as the dental drill. The display is displayed in real time, and is displayed in real time (e.g., refresh rate 30 to 60Hz) in accordance with movement of the manipulator 30 operated in the actual implant treatment by the practitioner.

Since the registration accuracy of the CT markers 11 of the plate 10 in real space and the CT markers 11 in the three-dimensional reconstructed image of the plate 10 in real space is ensured by the processing apparatus 300, positional accuracy of the image of the manipulator 30 superimposed on the three-dimensional reconstructed image satisfies the positional accuracy that the practitioner or the like requires. For this reason, the practitioner can start the dental implant treatments on a patient simply by confirming the registration accuracy using the recessed part of the plate 10 while he/she looks at the three-dimensional reconstructed image displayed on the display 60, without going through registration tasks which used to be carried out by the practitioner or the like prior to the treatments in conventional cases, and therefore it is possible to perform three-dimensional, intuitive operations. As described above, the surgical procedure support system 1 according to the embodiment can reduce the workload of the practitioner engaged in dental implant treatments.

<Functional configuration of each apparatus included in the surgical procedure support system 1>

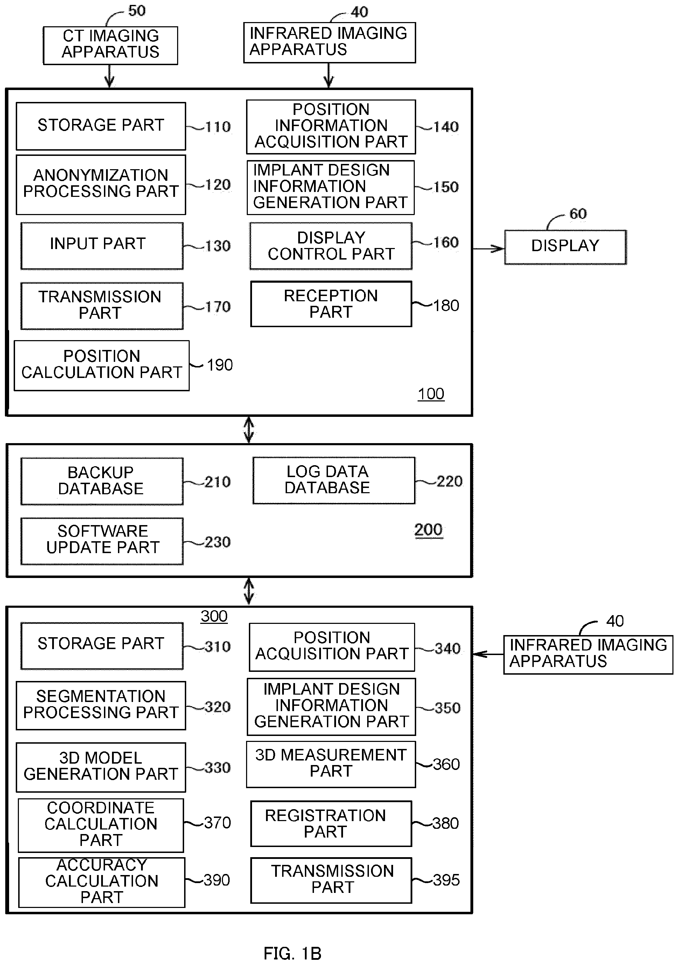

FIG. 1B shows an outline of functional blocks of the surgical procedure support apparatus 100, a management server 200, and a processing apparatus 300 implemented by the operation of hardware in accordance with software. The hardware of the surgical procedure support apparatus 100 and the processing apparatus 300 may be a general computer, and the hardware of the management server 200 may be a general server computer. The surgical procedure support apparatus 100 and the processing apparatus 300 may be configured to have the same function, thereby facilitating a batch update of the software of the apparatuses added to the surgical procedure support system 1. In this case, similar to the dental clinic where the surgical procedure support apparatus 100 is installed, a three-dimensional measurement part such as the infrared imaging apparatus 40 and a display device such as the display 60 are also installed in the remote support facility.

The surgical procedure support apparatus 100 includes the storage part 110 that stores various types of information such as CT image data (referred to as "DICOM data"); an anonymization processing part 120 that anonymizes information concerning the CT image; the input part 130 that accepts an input of various types of information such as information concerning a patient; the position information acquisition part 140 that specifies a spatial position of an infrared marker on the basis of image data of the infrared imaging apparatus 40 (see Patent Document 1); an implant design information generation part 150 that generates implant information (information such as a position to be implanted or type of implant) designed by a practitioner; the display control part 160 that displays various types of information including the three-dimensional reconstructed image on the display 60; the transmission part 170 that transmits, to the processing apparatus 300, DICOM data relating to the CT image; a reception part 180 that receives various types of information from the processing apparatus 300; and a position calculation part 190 that calculates a position of the manipulator 30 in the three-dimensional reconstructed image.

Here, the infrared markers 25 and 39 are made of a material that reflects infrared rays, and/or includes a light source that emits infrared rays (e.g., infrared light emitting diode). Incidentally, when the infrared markers 25 and 39 are made of the material that reflects infrared rays, a light source that radiates infrared rays to the infrared markers 25 and 39 may be included in the surgical procedure support system 1. The infrared imaging apparatus 40 includes a stereo infrared camera, for example, and can capture the infrared markers 25 and 39 from a plurality of different angles. When the infrared imaging apparatus 40 captures the dental drill with the drill blade as the manipulator 30, for example, the position information acquisition part 140 acquires position coordinates of a plurality of infrared markers 25 included in the reference frame 20 and position coordinates of a plurality of drill markers included in the drill reference frame having a plurality of drill markers. Further, the position calculation part 190 calculates a position of the drill blade in the three-dimensional reconstructed image on the basis of (i) the three-dimensional reconstructed image, (ii) the position coordinates of the plurality of infrared markers 25, (iii) the position coordinates of the plurality of drill markers, (iv) a relative positional relationship between the plurality of drill markers and the drill blade, and (v) a transformation matrix. By doing this, the display control part 160 can display, on the display 60, the drill blade superimposed on the 3D reconstructed image.

The management server 200 includes a backup database 210 that stores the various types of received data for backup, a log data database 220 that backs up log data such as a trajectory of the position of the manipulator 30 during the treatment, and a software update part 230 that keeps software for the surgical procedure support apparatus 100 and the processing apparatus 300 in the latest state.

The processing apparatus 300 includes a storage part 310 that stores various types of information including the DICOM data received from the surgical procedure support apparatus 100; a segmentation processing part 320 that performs coloring processing and correction processing of the CT image data received from the surgical procedure support apparatus 100 via the management server 200; a three-dimensional model generation part 330 that generates data of one or more three-dimensional reconstructed images on the basis of the segmented data; a position acquisition part 340 that accepts a designation of positions of a plurality of CT markers in the three-dimensional reconstructed image from a user of the processing apparatus 300; an implant design information generation part 350 that generates implant information (e.g., a position to be implanted or type of implant) designed by a dental technician; a three-dimensional measurement part 360 that measures the position coordinates of the plurality of infrared markers 25; a coordinate calculation part 370 that calculates the position coordinates of the plurality of CT markers 11 provided on the plate 10; a registration part 380 that implements the registration of the three-dimensional reconstructed image and a tooth-form plaster model; an accuracy calculation part 390 that calculates, as the registration accuracy, an error between the position of the recessed part and the position of the drill blade in the three-dimensional reconstructed image; and a transmission part 395 that transmits the three-dimensional reconstructed image, the positions of the plurality of CT markers 11 in the three-dimensional reconstructed image, and the transformation matrix to the surgical procedure support apparatus 100.

[Registration in the remote support facility]

More specifically, the storage part 310 acquires, from the surgical procedure support apparatus 100, DICOM data relating to the CT image captured while the stent, to which the plate 10 is fixed, is attached to the teeth of the patient and stores the DICOM data. The three-dimensional model generation part 330 generates the three-dimensional reconstructed CT image on the basis of the DICOM data. The position acquisition part 340 receives a designation of the positions of the plurality of CT markers 11 in the three-dimensional reconstructed images generated by the three-dimensional model generation part 330 from the user of the processing apparatus 300 via a pointing device (not shown) such as a mouse included in the processing apparatus 300.

The three-dimensional measurement part 360 measures the position coordinates of the plurality of infrared markers 25 provided on the reference frame 20 in a state where the stent, to which the plate 10 connected with the reference frame 20 is fixed, is mounted on the tooth-form plaster model of the patient. The three-dimensional measurement part measures the respective position coordinates on the basis of an image captured by the infrared imaging apparatus 40, for example. The coordinate calculation part 370 calculates the position coordinates of the plurality of CT markers 11 provided on the plate 10 on the basis of the relative positional relationship between the position coordinates of the plurality of infrared markers 25 and the plurality of CT markers 11. The registration part 380 implements the registration of the three-dimensional reconstructed image and the tooth-form plaster model on the basis of the positions of the plurality of CT markers 11 in the three-dimensional reconstructed image designated by the user and the position coordinates of the CT markers 11 in real space calculated by the coordinate calculation part 370. As a result, even the user in the remote support facility can accurately align the three-dimensional reconstructed image and the tooth-form plaster model.

[Details of the plate 10 and the reference frame 20]

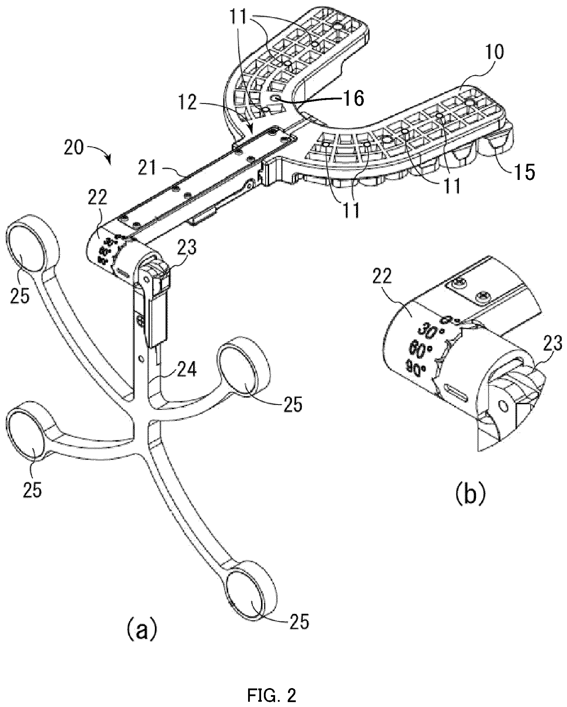

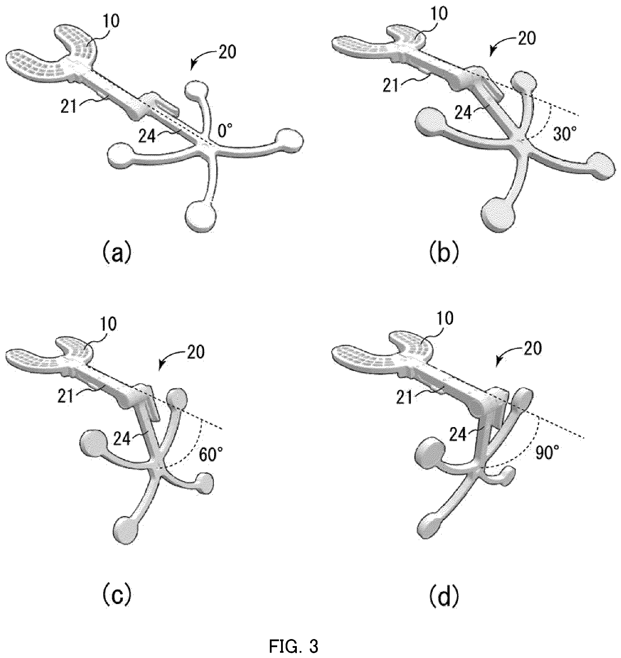

FIGS. 2A, 2B, and FIGS. 3A to 3D are each a schematic diagram of the plate 10 and the reference frame 20. Hereinafter, the plate 10 and the reference frame 20 used in the surgical procedure support system 1 according to the embodiment will be described in more detail. Note that FIG. 2A shows the plate 10 in a state of being mounted on a stent 15 (a mouth-piece-like object formed of a transparent resin) made from the tooth-form plaster model of the patient. In other words, the plate 10 according to the embodiment is used by being fixed to the stent 15 which is to be mounted on the row of teeth of the patient.

In the plate 10, the plurality of CT markers 11 made of metal, e.g. micro-balls made of titanium, are embedded such that the CT markers 11 are clearly distinguishable on a CT image. The plate 10 also includes a detachable part 12 for connecting the reference frame 20 that includes a plurality of infrared markers 25 whose relative positional relationship with each of the plurality of CT markers 11 when the reference frame is connected to the plate 10 is known. In the example shown in FIG. 2A, the plate 10 is mounted on the detachable part 12 with a first frame 21 of the reference frame 20. The number of CT markers 11 is at least three. Note that a preferred embodiment in which the number of CT markers 11 is seven will be described in this specification, but the present disclosure is not limited to this.

The plate 10 further includes a recessed part 16 to be used in measuring registration accuracy between (i) the position coordinates of the plurality of CT markers 11 in the three-dimensional reconstructed CT image including the plate 10 as the object and (ii) position coordinates of the CT markers 11 in real space in which the plate 10 exists, the position coordinates being calculated on the basis of the position coordinates of the plurality of infrared markers 25 included in the reference frame 20. As described above, in the remote support facility, the user of the processing apparatus 300 can confirm the registration accuracy of the CT markers 11 of the plate 10 in real space and the CT markers 11 in the three-dimensional reconstructed image by using the tooth-form plaster model of the patient and the recessed part 16 of the plate 10, even at a place where the practitioner is not present. Therefore, the user in the remote support facility can secure the registration accuracy by repeatedly implementing the registration until the positional accuracy required by the practitioner or the like is reached.

The reference frame 20 includes a first frame 21 and a second frame 24, which is connected to the first frame 21 at a connection part 22 and has a plurality of infrared markers 25. The number of the infrared markers 25 may be three or more, and as the number increases, accuracy of the position measurement increases, but the amount of data to be processed increases. Note that, in this specification, the reference frame 20 provided with 4 infrared markers 25 is described as a preferred embodiment, but the present disclosure is not limited to this.

The connection part 22 is configured such that a rotational position of a second frame 24 with respect to a first frame 21 (and the plate 10) is limited to a plurality of predetermined angles, and the first frame 21 and the second frame 24 can be locked by a locking mechanism 23 (FIG. 2B). In other words, it is configured so that the first frame 21 and the second frame 24 cannot be fixed at any angle other than the predetermined angles, and the reference frame 20 rotates only to the plurality of predetermined angles with respect to the plate fixed to the stent 15 (the stent 15 mounted on the tooth-form plaster model). For example, as shown in FIGS. 3A to 3D, the plurality of predetermined angles are four levels, and the angle between the first frame 21 and the second frame 24 is configured to be any of 0°, 30°, 60°, and 90°. In this specification, a preferable embodiment in which the predetermined angles between the first frame 21 and the second frame 24 are 0°, 30°, 60°, and 90° is described, but the present disclosure is not limited to this.

In CT imaging, the reference frame 20 is removed from the plate 10 and the plate 10 mounted on the stent is fixed to the patient's teeth. On the other hand, designing of the dental implant by the practitioner and/or the dental technician and the dental implant treatments by the practitioner are performed with the reference frame 20 attached to the plate 10.

[Generation and transmission of the transformation matrix]

With reference to FIG. 1B again, the registration part 380 of the processing apparatus 300 generates the transformation matrix for transforming the positions of the plurality of infrared markers 25 provided in the reference frame 20 into the positions of the plurality of CT markers 11 in the three-dimensional reconstructed image for each predetermined angle, which is an angle that the first frame 21 and the second frame 24 can take, on the basis of (i) known design information regarding the relative positional relationship between the plurality of infrared markers 25 provided in the reference frame 20 and the plurality of CT markers 11 provided in the plate 10 and (ii) the positions of the plurality of CT markers 11 in the three-dimensional reconstructed image.

By doing this, when the reference frame 20 becomes an obstacle to the practitioner during the treatment of the implant, such an issue can be solved by changing the angle. Further, by using the transformation matrix corresponding to the changed angle, the positional relation between (i) position and orientation of the manipulator 30 on the display 60 and (ii) the three-dimensional reconstructed image of the plate 10, the teeth, the lower jawbone, and the like of the patient can be maintained normally. It should be noted that the known design information regarding the relative positional relationship between the plurality of infrared markers 25 provided in the reference frame 20 and the plurality of CT markers 11 provided in the plate 10 is stored in advance in the storage part 310.

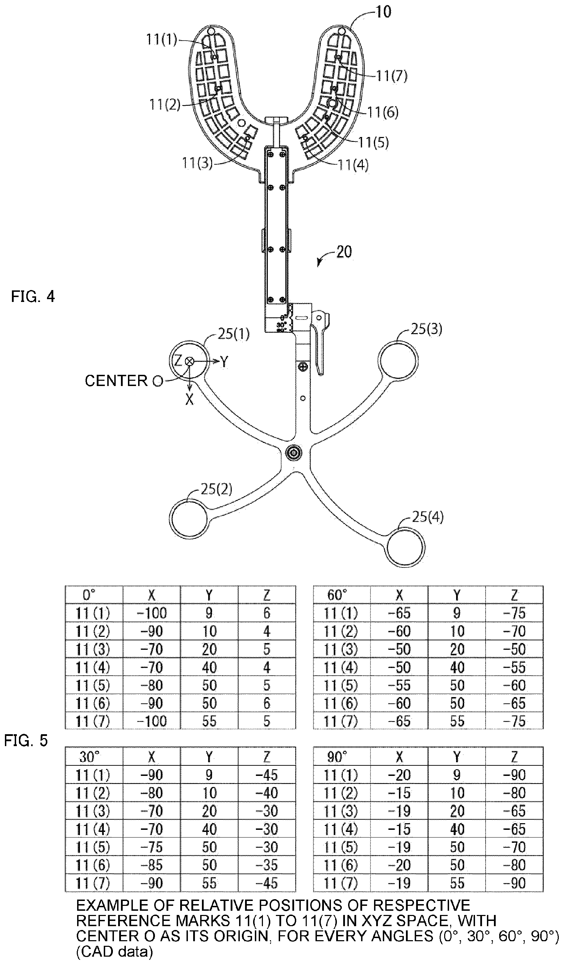

FIG. 4 shows an illustrative example of a generation of a transformation matrix. Considering an XYZ space with the center O of 25(1), one of four predetermined infrared markers 25, as its origin, as shown in FIG. 4, the respective positions (X, Y, Z) of seven CT markers 11(1) to 11(7) of the plate 10 with respect to the center O can be determined in advance at the design stage of the plate 10 and the reference frame 20. Further, the positions (X, Y, Z) of the seven CT markers 11(1) to 11(7) change according to the predetermined angles (0°, 30°, 60°, and 90°) between the first frame 21 and the second frame 24 of the reference frame 20, and they can also be determined in advance.

FIG. 5 shows an example of the known design information. Specifically, FIG. 5 is an example of the positions (X, Y, Z) of the seven CT markers 11(1) to 11(7) of the plate 10 with respect to the center O, determined in advance, for every predetermined angle (0°, 30°, 60°, and 90°). Such information (CAD data) of the relative position of the CT markers 11 with respect to the center O is stored in advance in the surgical procedure support apparatus 100 and/or the processing apparatus 300.

The registration part 380 reads the CAD data of the CT markers 11 from the storage part 310 and generates a transformation matrix [R, t] for deriving the positions of the CT markers 11 in the CT image from the positions of the infrared markers 25 on the basis of the CAD data (see below equation).

$$A = R*B+t$$

In the equation, A is a matrix of the positions (x, y, z) of the CT markers 11 in the three-dimensional reconstructed image, B is a matrix of CAD data of the CT markers 11 (positions (X, Y, Z) of the CT markers 11 with respect to the center O), R is a rotation matrix, and t is a translation matrix. Since the value of B for each predetermined angle is different, the registration part 380 obtains the transformation matrix [R, t] for each predetermined angle.

The transmission part 395 transmits the three-dimensional reconstructed image, the positions of the plurality of CT markers 11 in the three-dimensional reconstructed image, and the transformation matrix for each predetermined angle to the surgical procedure support apparatus 100, which is a transmission-source device, from which DICOM data is transmitted via the network. As a result, the dentist or the like who is the user of the surgical procedure support apparatus 100 can quickly start the treatment on the basis of information received from the transmission part 395.

As described above, by using the surgical procedure support system 1 according to the present embodiment, processing such as the segmentation processing and the registration processing, which have been conventionally performed in the dental clinic, can be performed by a professional dental technician or the like who is present remotely and uses the same system. Therefore the work efficiency of the practitioner is improved and the workload of the practitioner is reduced. In addition, the practitioner can easily select a remote support facility or the like to which he/she commissions various processes in consideration of his/her preference, congestion situations, or the cost of the remote support facility or the like. In addition, since various types of data passing through the management server 200 are saved for backup, it is possible to reduce the risk when the practitioner accidentally loses or deletes the data.

[Example of the manipulator 30]

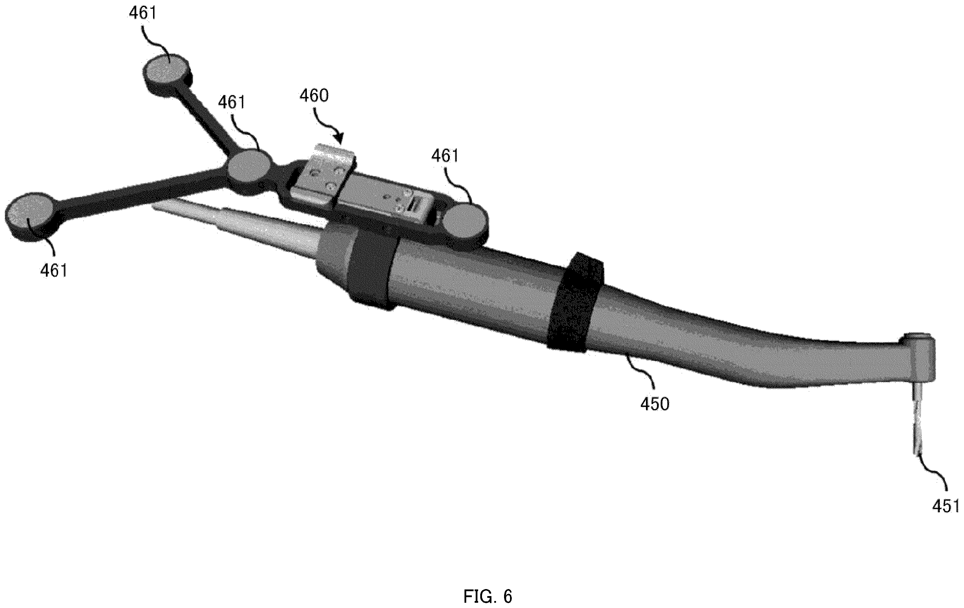

FIG. 6 is a schematic diagram of the manipulator 30 used by the practitioner in the treatment and/or designing of a dental implant, and shows, as an example of various existing manipulators 30, a drill 450 having a drill blade 451. The drill 450 is a dental drill having the drill blade 451 attached to its distal end portion and a drill frame 460 detachably attached to its end portion. The drill frame 460 includes arms extending in three directions from its tip, and a disc-shaped drill marker 461 is attached to the tip of each arm. The drill marker 461 is made of an infrared reflective material and an infrared light emitting diode (IR LED). The drill blade 451 cuts the gums and bones of the patient while rotating with a direction approximately perpendicular to the longitudinal direction of the drill 450 as an axis. The drill 450 is attached when cutting the gums and bones, but an artificial tooth root, instead of the drill 450, is attached when embedding the artificial tooth root in the cut hole.

Each drill marker 461 is provided at the fixed position with respect to the drill 450, and once the position of each drill marker 461 is specified, it is possible to specify the position of the tip of the drill 450 and the positions of parts such as the drill blade 451 (or the artificial tooth root) to be attached thereto. Therefore, when the relative positional relationship between the drill marker 461 of the drill 450 and the infrared marker 25 of the reference frame 20 is specified, it is possible to align the positions of the parts such as the drill 450 with the position of the teeth, jaws, or the like of the patient.

In design work of the implant using the manipulator 30 such as the drill 450, a plaster model simulating the patients' teeth and jaws may be used, or the patient's teeth and jaws themselves may be used. Also, in the design work, a part for designing (for example, the parts described in FIG. 6 of the above-mentioned Patent Document 1) may be used by attaching it at the tip of the manipulator 30.

[Confirming the registration accuracy]

With reference to FIG. 1B once again, the three-dimensional measurement part 360 captures the drill frame 460 having the plurality of drill markers 461, using the infrared imaging apparatus 40 or the like. The drill frame 460 is attachable and detachable to and from the drill 450 having the drill blade 451, and its relative positional relationship with the drill blade 451 is known.

The registration part 380 calculates a position of the drill blade 451 in the three-dimensional reconstructed image on the basis of (i) the position coordinates of the plurality of drill markers 461 measured by the three-dimensional measurement part 360, (ii) the relative positional relationship between the plurality of drill markers 461 and the drill blade 451, (iii) the position coordinates of the plurality of infrared markers 25, and (iv) the relative positional relationship between the position coordinates of the plurality of infrared markers 25 and the plurality of CT markers 11. The accuracy calculation part 390 calculates, as the registration accuracy, the error between the position of the recessed part 16 and the position of the drill blade 451 in the three-dimensional reconstructed image when the user of the processing apparatus 300 inserts the drill blade 451 into the recessed part 16 of the plate 10. The transmission part 395 further transmits the registration accuracy calculated by the accuracy calculation part 390 to the surgical procedure support apparatus 100, which is a transmission-source device, from which DICOM data is transmitted via the network. As a result, the user, such as a dentist, of the surgical procedure support apparatus 100 can grasp at a glance the registration accuracy of the CT marker 11 received from the processing apparatus 300, without examining the registration accuracy by himself/herself.

<Process flow>

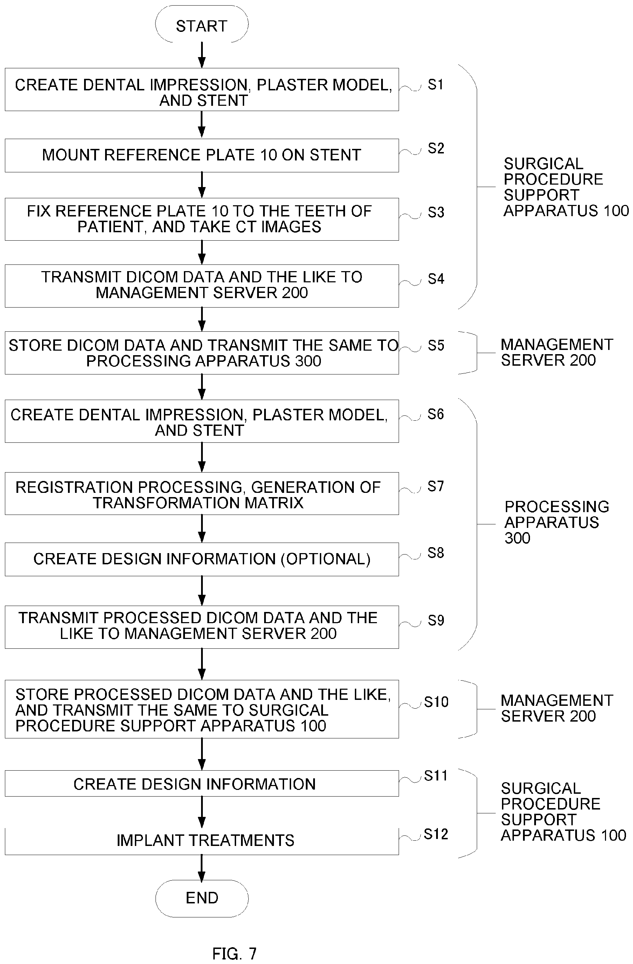

Next, referring to FIGS. 7 to 10, an outline of a flow of a dental implant treatment using the surgical procedure support system 1 according to the present embodiment will be described using a specific example, as appropriate.

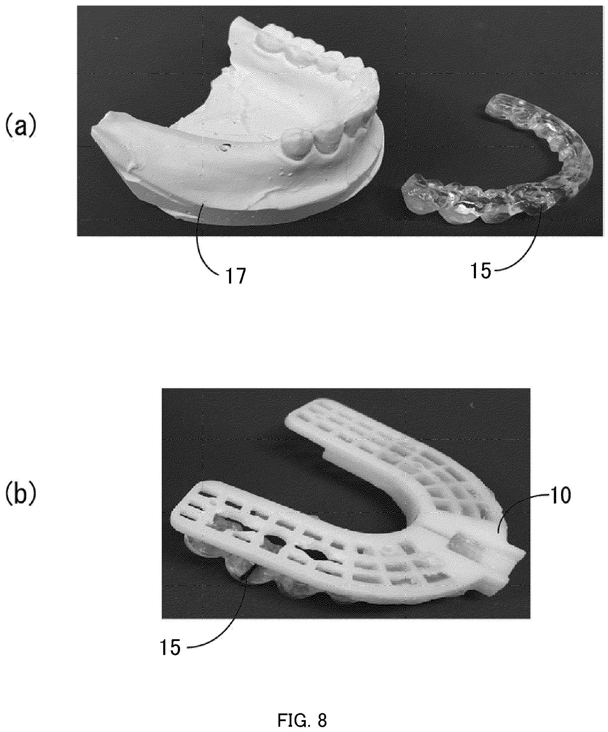

First, in step S1, a practitioner (dentist) who has received a request to perform an implant in a dental clinic takes a dental impression of a patient, creates a plaster model 17 which is a tooth-form plaster model, and creates a stent 15 (mouth piece) from the plaster model 17. For example, FIG. 8A shows an example of a plaster model 17 and an example of the stent 15. It should be noted that these processes are pre-processes of implant treatments that have been carried out conventionally.

In step S2, the practitioner prepares a plate 10 and attaches the plate 10 to the stent 15 (FIG. 8B). The plate 10 is firmly secured to the stent 15 such that it does not slip out of place.

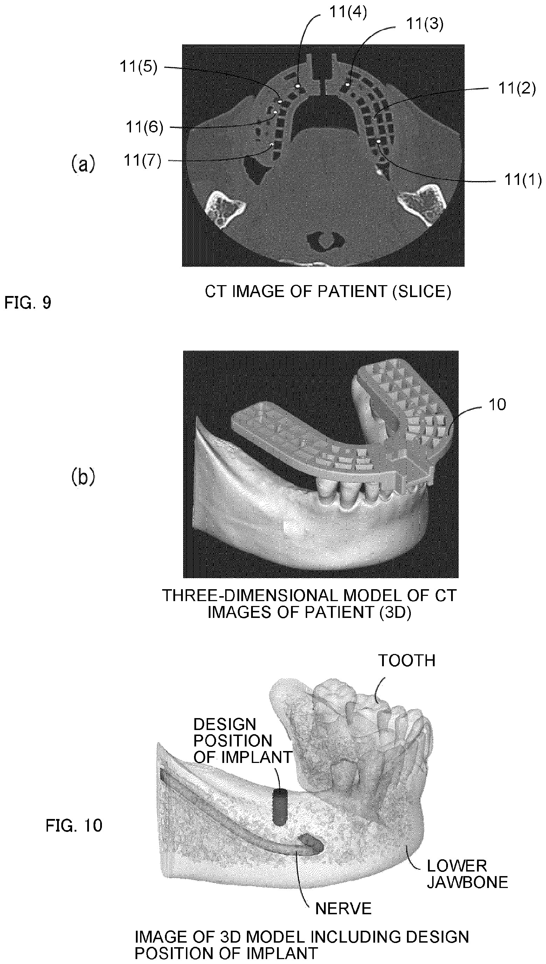

In step S3, the practitioner fixes the plate 10, which is attached to the stent 15, to the teeth of the patient, and takes CT images (several hundreds of slice images) using the CT imaging apparatus 50. FIG. 9A is an example of a CT image (a single slice image) of the patient, and it can be seen that seven CT markers 11(1) to 11(7) are clearly appearing in the image in a form that can be distinguished from other parts such as the bones. FIG. 9B shows an example of a three-dimensional reconstructed image of the plate 10 and the patient generated from the CT images.

In step S4, the surgical procedure support apparatus 100 receives the DICOM data of the CT images of the patient from the CT imaging apparatus 50, stores the DICOM data as original data in the storage part 11, as appropriate, and transmits the DICOM data to the management server 200 via the network. At this time, it is preferable that the DICOM data is anonymized by the anonymization processing part 120 of the surgical procedure support apparatus 100 so that a relationship between the patient and the DICOM data is not known to a third party. Further, together with the DICOM data, (i) ID information concerning a remote support facility to be designated so that the management server 200 can specify the destination, (ii) ID information concerning the dental clinic of the practitioner, (iii) an ID number of the patient, and optionally (iv) implant information (information concerning a site, or the like, where an implant is to be embedded) are transmitted from the surgical procedure support apparatus 100 to the management server 200. Hereinafter, the ID information concerning the remote support facility, the ID information concerning the dental clinic of the practitioner, the ID number of the patient, and optionally the implant information are referred to as "DICOM data and the like".

In step S5, the management server 200 stores the received DICOM data and the like in the backup database 210, and transmits the DICOM data and the like to the processing apparatus 300 of the designated remote support facility or the like. The management server 200 can store the DICOM data and the like in the backup database 210 for backup, and provide this data in response to a request from the practitioner.

In step S6, the dental technician or the like of the remote support facility uses the segmentation processing part 320 of the processing apparatus 300 to perform segmentation processing, that is, to color the received DICOM data for each part and correct unclear part, as appropriate. Thereafter, the three-dimensional model generation part 330 of the processing apparatus 300 generates one or more three-dimensional reconstructed images using the processed data. For example, the dental technician colors the parts on the image data corresponding to the bones, the nerves, and the CT markers 11 with different colors (in particular, it is advisable to paint the nerves with a highly prominent color since they should not be damaged) and corrects a part that is blurred due to halation or the like, as appropriate. Further, the dental technician specifies position information (x, y, z) in the three-dimensional reconstructed images of the CT markers 11 and stores the position information (x, y, z) in the storage part 310 of the processing apparatus 300 in association with data of the three-dimensional reconstructed images. Note that these tasks may be performed automatically by the processing apparatus 300, or may be conducted manually under visual observation by the dental technician or the like.

In step S7, the registration part 380 of the processing apparatus 300 generates, for each of the predetermined angles between the first frame 21 and the second frame 24 of the reference frame 20, a transformation matrix for determining the positions of the CT markers 11 in the three-dimensional reconstructed image on the basis of the infrared markers 25.

In step S8, optionally, the dental technician considers a recommended design for the dental implant and creates information concerning the recommended design (information such as a position and type of the implant).

In step S9, the processing apparatus 300 transmits the information concerning the three-dimensional reconstructed images and the transformation matrix, the ID information concerning the dental clinic, or the like to the management server 200 via the network.

In step S10, the management server 200 stores the information concerning the three-dimensional reconstructed images and the transformation matrix or the like in the backup database 210, and transmits the information to the surgical procedure support apparatus 100 of the original dental clinic. Note that the management server 200 may store the information concerning the three-dimensional reconstructed images and the transformation matrix or the like for backup, and provide the information in response to a request from the practitioner.

In step S11, the practitioner designs the implant (determines the position, type, and the like of the implant) in the three-dimensional reconstructed image on the basis of the information concerning the three-dimensional reconstructed images and the transformation matrix or the like, using the implant design information generation part 150 of the surgical procedure support apparatus 100, and stores the design information in the storage part 110. FIG. 10 shows an example of the design position of the implant in the three-dimensional reconstructed image.

In step S12, the practitioner fixes the plate 10 on which the reference frame 20 is mounted to the patient's teeth, and sets the angle between the plate 10 and the reference frame 20 to the predetermined angle (any one of 0°, 30°, 60°, and 90°). By selecting the transformation matrix corresponding to the angle and positioning, on the display 60, the drill blade 451 at the tip of the drill 450 to the designed implant position while looking at the three-dimensional reconstructed image displayed on the display 60, the practitioner can accurately perform the implant treatment at the actual designed position. Since the relative positional relationship between the CT markers 11 and the infrared markers 25 and the relative positional relationship between the infrared markers 25 and the drill markers 461 are accurately reflected on the display 60 in real time, the practitioner can perform the implant treatment while looking at the display 60.

<Example of a display screen>

Next, a display screen to be displayed on the display 60, which is a display device, by the display control part 160 of the surgical procedure support apparatus 100 according to the embodiment will be described.

[Implant-implantation-support screen]

The display control part 160 causes the display 60, functioning as a display part of the surgical procedure support apparatus 100, to display (1) an image extracted from the three-dimensional reconstructed image and including the bones into which an implant of the patient is to be implanted, (2) a predetermined implantation completion position of the implant, (3) a predicted implantation completion position of the implant calculated on the basis of a current position of the drill blade 451 calculated by the position calculation part 190, and (4) a current position of the implant corresponding to the current position of the drill blade 451, in different modes.

More detailed descriptions are given below. In response to a signal received from the processing apparatus 300, the display control part 160 displays the bones and teeth, obtained from CT data, into which the implant is to be embedded, and displays the predetermined implantation completion position of the implant in a first attribute, e.g., in red, the predicted implantation completion position of the implant in a second attribute, e.g., in yellow, and the current position of the implant in a third attribute, e.g., in blue.

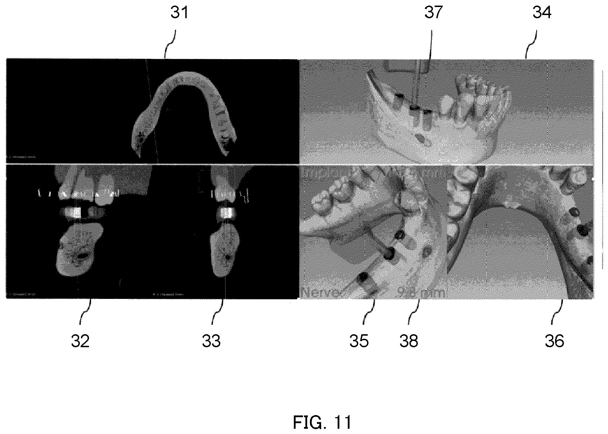

Using FIG. 11, an implant-implantation-support screen will be described. The image is divided into six regions. Regions 31 to 33 on the left display three multisection reconstructed images (MPR image, multi-planar reconstruction, multi planar reconstruction) based on CT data, and regions 34 to 36 on the right show three 3D rendered images based on CT data. As the multi-section reconstructed images and the 3D rendered images, images viewed from three different directions from among above, outside, inside, bottom, and the like can be appropriately displayed according to the practitioner's needs.

For example, in the region 31, a cross section specified by the approximately horizontal line of the cross-hairs shown in the regions 32 and 33 is displayed, in the region 32, a cross section specified by the approximately vertical line of the cross-hairs shown in the regions 31 and 33 is displayed, and in the region 33, a cross section specified by the approximately horizontal line shown in the region 31 and the approximately vertical line shown in the region 32 is displayed. These approximately horizontal lines and approximately vertical lines are colored with the same color corresponding to each cross section. Further, in the region 35, a distance 37 which is between the bottom of the implantation completion position and the tip of the drill blade 451, and a distance 38 which is between the nerve and the tip of the drill blade 451 are displayed. As a result, the practitioner can grasp the specific numerical values and perform tasks accurately.

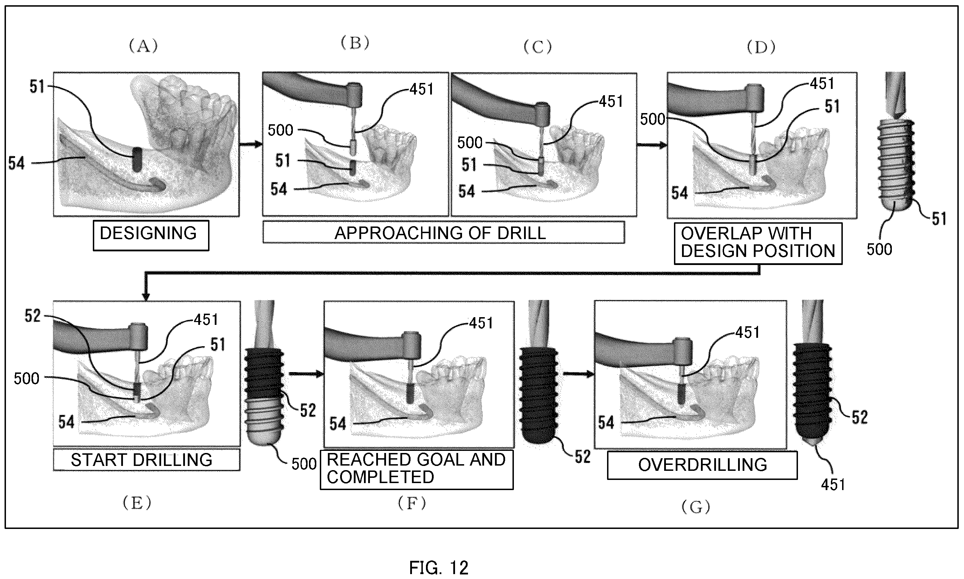

Next, a procedure process of an implant using the surgical procedure support system 1 will be described using FIG. 12. The procedure process includes a design process, an approach process, and a cutting process.

The design process is a process of determining the position of the implant on the basis of the CT data. First, on the basis of the CT data, the practitioner determines the position of a nerve 54 present in the bones. Then, the practitioner specifies the position of the implant so as not to overlap with the position of the nerve 54 and in consideration of various circumstances. The practitioner inputs data concerning the positions of the nerve 54 and the implant to the surgical procedure support apparatus 100.

The approach process is a process of determining an entry angle of the drill blade 451 with respect to the bones. First, the surgical procedure support apparatus 100 calculates (i) the bones and teeth into which the implant is to be embedded, (ii) an implantation completion position 51 of the implant determined in advance, and (iii) the position of the nerve 54, and generates a composite image of the bones and teeth, the implantation completion position 51, and the nerve 54. The display 60 then displays the composite image (see (A) in FIG. 12). At this time, the implantation completion position 51 is displayed in red. Next, the practitioner introduces the drill blade 451 into the oral cavity of the patient while referring to the image displayed on the display 60, and temporarily places the drill blade 451 at a position where the implant is to be provided. At this time, the infrared imaging apparatus 40 continuously transmits images obtained by capturing the infrared markers 25 and the drill markers 461 provided on the reference frame 20 to the surgical procedure support apparatus 100.

The surgical procedure support apparatus 100 calculates a position of the drill blade 451 in the oral cavity using the images. On the basis of the obtained position, the surgical procedure support apparatus 100 causes the display 60 to continuously display the position of the drill blade 451 with respect to the bones, together with a virtual implant 500 virtually added to the tip of the drill blade 451 (see (B) in FIG. 12). The virtual implant 500 indicates the predicted implantation completion position for predicting a position where the implant is to be embedded, and is displayed in yellow. When the practitioner operates the dental drill to change the position and angle of the drill blade 451 with respect to the bones, the positions and angle of the drill blade 451 and the virtual implant 500 displayed on the display 60 change in accordance with the change (see (C) and (D) in FIG. 12). When the practitioner confirms that the position and the angle of the virtual implant 500 match the implantation completion position 51, the process proceeds to the following cutting process.

The cutting process is a process of actually drilling a hole in the bones of the patient. Following the approach process, the practitioner operates the drill blade 451 and starts cutting (see (E) in FIG. 12) while maintaining a state where the position and angle of the virtual implant 500 match the implantation completion position 51. In this process, in a similar manner as with the approach processing, the infrared imaging apparatus 40, the surgical procedure support apparatus 100, and the display 60 continuously operate, and the position of the drill blade 451 entering in the bones is continuously displayed on the display 60 in blue as the current position 52. Since the respective positions are continuously updated and displayed, when the current position 52 deviates from the positions and angles determined in the approach process, the virtual implant 500 deviates from the implantation completion position 51 in the display 60. Therefore, the practitioner who sees such a deviation can easily perceives that if he/she continues the cutting at the current angle, a hole will be formed at a position deviated from the implantation completion position 51, and can correct the entry angle to the appropriate angle. Then, the practitioner performs the cutting until the current position 52 matches the implantation completion position 51 (see (F) in FIG. 12), and completes the cutting process.

It should be noted that if the current position 52 exceeds the implantation completion position 51, the drill blade 451 protrudes from the bottom of the implantation completion position 51 (see (G) in FIG. 12). As a result, the practitioner can easily perceive that the current position 52 exceeds the implantation completion position 51, and can stop the cutting. According to the above, the practitioner can perform, while looking at the image displayed on the display 60, the cutting while grasping and correcting the position and angle of the drill blade 451.

[Depth marker]

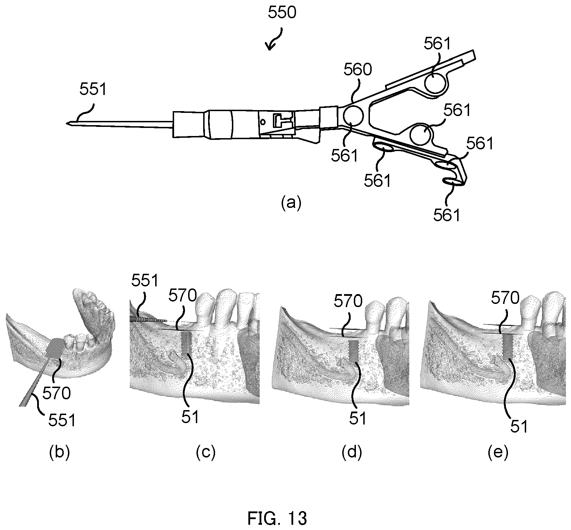

FIGS. 13A to 13E each schematically show a tool for supporting a vertical-position designing of the implant. The vertical position of the implant is one of the factors that greatly affects the aesthetics of a superstructure of the implant. Controlling the vertical position together with a buccolingual position is essential to obtain an aesthetic treatment result, and a display screen relevant to a depth marker which is displayed by the display control part 160 on the display 60 supports the controlling of such a position.

FIG. 13A schematically shows a pointing tool 550, which is a type of the manipulator 30 for supporting the vertical position. The pointing tool 550 includes a pointing part 551 for pointing out a point in real space, and a tool frame 560 having a plurality of markers 561 for the pointing part (hereinafter, pointing markers 561). The pointing markers 561 are detachable from the pointing part 551, and their relative positional relationship with the pointing part 551 is known.

The practitioner points out a gingival margin line desired to be restored with the pointing part 551 of the pointing tool 550 at the time of position-designing in the plaster model 17 or the oral cavity of the patient. At this time, the position information acquisition part 140 acquires position coordinates of the plurality of pointing markers 561 included in the tool frame 560 in addition to the position coordinates of the infrared markers 25 included in the reference frame 20. The position calculation part 190 calculates a position of the pointing part 551 in the three-dimensional reconstructed image on the basis of the three-dimensional reconstructed image, the position coordinates of the plurality of infrared markers 25, the position coordinates of the plurality of pointing markers 561, a relative positional relationship between the plurality of pointing markers 561 and the pointing part 551, and the transformation matrix.

The display control part 160 superimposes a virtual plane 570 on the image including (i) the bones into which the implant of the patient is to be embedded and (ii) the predetermined implantation completion position 51, and displays the superimposed image on the display 60 (see FIGS. 13B to 13E). The virtual plane 570 is a plane parallel to a plane whose normal is the longitudinal direction of the predetermined implantation completion position 51 of the implant in the three-dimensional reconstructed image. A distance between the virtual plane 570 and the position of the pointing part 551 in the three-dimensional reconstructed image is a predetermined distance.

In FIGS. 13B and 13C, a gap occurs between the virtual plane 570 and the front surface of the implantation completion position 51. This suggests that the vertical position of the implant is not at a proper position from an aesthetic point of view. Therefore, as shown in FIG. 13E, the practitioner moves the implantation completion position 51 such that the front surface of the implantation completion position 51 matches the virtual plane 570. By doing this, the practitioner can cause the vertical position of the implant to be the proper position during a planning stage prior to an insertion of the implant. The distance between the position of the pointing part 551 and the plane 570 in the three-dimensional reconstructed image is 3 millimeters by default.

[Orthodontic anchor screw navigation]

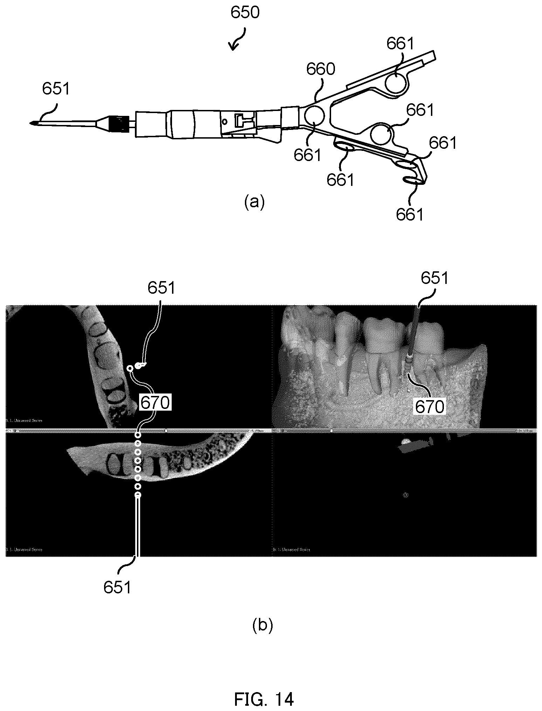

FIGS. 14A and 14B each schematically show a tool for an orthodontic anchor screw navigation. The orthodontic anchor screw navigation is a display screen intended to present the anatomical positional relationship between the orthodontic anchor screw and the natural tooth root, maxillary sinus, or the like to the practitioner in real time when the orthodontic anchor screw is implanted in the oral cavity of the patient, thereby making it easier to embed and install the orthodontic anchor screw in a safe site.

FIG. 14A schematically shows a driver tool 650, which is a type of the manipulator 30, for implanting the orthodontic anchor screw. The driver tool 650 includes a driver frame 660 having (i) a driver 651 to be used to implant the orthodontic anchor screw and (ii) a plurality of markers 661 for the driver (hereinafter, driver markers 661). The driver markers 651 are detachable to the driver 651, and their relative positional relationship with the driver 651 is known.

The practitioner uses the driver 651 to which the driver frame 660 is connected when the orthodontic anchor screw is implanted and installed in the oral cavity of the patient. At this time, the position information acquisition part 140 acquires position coordinates of the plurality of driver markers 661 included in the driver frame 660 in addition to the position coordinates of the infrared markers 25 included in the reference frame 20. The position calculation part 190 calculates a position of the driver 651 in the three-dimensional reconstructed image on the basis of the three-dimensional reconstructed image, the position coordinates of the plurality of infrared markers 25, the position coordinates of the plurality of driver markers 661, a relative positional relationship between the plurality of driver markers 661 and the driver 651, and the transformation matrix.

The display control part 160 superimposes a virtual image 670, in which the driver 651 is advanced along the long axis of the driver 651, on the image including the bones in which the orthodontic anchor screw of the patient is to be embedded, and displays the superimposed image on the display 60 (see FIG. 14B).

By looking at the display 60 displaying the display screen showing the anchor screw navigation shown in FIG. 14B, the practitioner can grasp at a glance the anatomical positional relationship between the orthodontic anchor screw and the natural tooth root, maxillary sinus, or the like. Therefore, the surgical procedure support apparatus 100 can provide support to the practitioner to implant and install the orthodontic anchor screw in a safe site in the oral cavity of the patient.

While the foregoing description is directed to one embodiment of the present disclosure, it should be noted that other variations and modifications are obvious to those skilled in the art and may be made without departing from the scope and spirit of the disclosure.

The present disclosure may be specified by the items shown below.

[1-1]

A surgical procedure support system (1) for a dental implant, the system including: a surgical procedure support apparatus (100) at a first facility where dental implant treatments are performed; a management server (200) at a second facility different from the first facility, the management server (200) being connected to the surgical procedure support apparatus (100) via a network; and a processing apparatus (300) at the second facility or at a third facility which is different from the first facility and the second facility, the processing apparatus (300) being connected to the management server (200) via the network, wherein the surgical procedure support apparatus (100) transmits, to the management server (200), DICOM data relating to a CT image obtained in a state where a plate (10) embedded with a plurality of CT markers (11) is attached to the teeth of a patient, the management server (200) transmits the DICOM data to the processing apparatus (300), the processing apparatus (300) processes the DICOM data, generates one or more three-dimensional reconstructed images of the CT image, and transmits data of the one or more three-dimensional reconstructed images to the management server (200), and the management server (200) transmits the data of the one or more three-dimensional reconstructed images to the surgical procedure support apparatus (100) .

[1-2]

The surgical procedure support system according to [1-1], wherein a reference frame (20, 24) to be mounted on the plate (10) rotates at a plurality of predetermined angles with respect to the plate (10), and the processing apparatus (300) generates a transformation matrix for generating position information concerning the CT markers (11) in the one or more three-dimensional reconstructed images for the each of the predetermined angles on the basis of (i) known design information regarding a relative positional relationship between a plurality of infrared markers (25) provided in the reference frame (20) and the CT markers (11) and (ii) positional information concerning the CT markers (11) in the one or more three-dimensional reconstructed images, and transmits the transformation matrix to the management server together with the data of the one or more three-dimensional reconstructed images.

[1-3]

The surgical procedure support system according to [1-1] or [1-2], wherein the processing of the DICOM data by the processing apparatus (300) includes coloring processing of the CT image data.

[1-4]

A method for supporting surgical procedure of a dental implant in a surgical procedure support system (1) which includes a surgical procedure support apparatus (100) at a first facility where dental implant treatments are performed, a management server (200) at a second facility different from the first facility, the management server (200) being connected to the surgical procedure support apparatus via a network, a processing apparatus (300) at the second facility or at a third facility different from the first facility and the second facility, the processing apparatus (300) being connected to the management server (200) via the network, the method for supporting surgical procedure includes: a step in which the surgical procedure support apparatus (100) transmits, to the management server (200), DICOM data relating to a CT image obtained in a state where a plate (10) embedded with a plurality of CT markers (11) is attached to the teeth of a patient; a step in which the surgical procedure support apparatus (100) transmits the DICOM data to the processing apparatus (300); a step in which the processing apparatus (300) processes the DICOM data, generates one or more three-dimensional reconstructed images of the CT image, and transmits data of the one or more three-dimensional reconstructed images to the management server (200); and a step in which the management server (200) transmits the one or more three-dimensional reconstructed images to the procedure support apparatus (100).

[1-5]

The method for supporting surgical procedure according to [1-4], wherein a reference frame (20, 24) to be mounted on the plate (10) rotates at a plurality of predetermined angles with respect to the plate (10), and the processing apparatus (300) generates a transformation matrix for generating position information concerning the CT markers (11) in the one or more three-dimensional reconstructed images for the each of the predetermined angles on the basis of (i) known design information regarding a relative positional relationship between a plurality of infrared markers (25) provided in the reference frame (20) and the CT markers (11) and (ii) positional information concerning the CT markers (11) in the one or more three-dimensional reconstructed images, and transmits the transformation matrix to the management server together with the data of the one or more three-dimensional reconstructed images.

[1-6]

The method for supporting surgical procedure according to [1-4] or [1-5], wherein the processing of the DICOM data by the processing apparatus (300) includes coloring processing of the CT image data.

[2-1]

A display device for surgical procedure support that displays the bones into which an implant is to be embedded, displays a predetermined implantation completion position of the implant in a first attribute, displays a predicted implantation completion position of the implant calculated on the basis of a current position of a drill blade in a second attribute, and displays a current position of the implant corresponding to the current position of the drill blade in a third attribute.

[2-2]

The display device for surgical procedure support described in [2-1] displays the nerves present in the bones.

[2-3]

The display device for surgical procedure support described in [2-2] displays a distance between the nerves and a current tip position of the drill blade.

[2-4]

The display device for surgical procedure support described in any one of [2-1] to [2-3] displays a distance between the deepest part of the implantation completion position and the current tip position of the drill blade.

[2-5]

The display device for surgical procedure support described in any one of [2-1] to [2-4] displays images of the bone, the implantation completion position, the predicted implantation completion position, and the current position that are viewed from three different directions.

[2-6]

A surgical procedure support system including: a drill frame having a drill marker and detachably attached to a dental drill having a drill blade; a fixed frame having a marker for fixed frame (fixed frame marker) and being mounted such that it extends out of the oral cavity from a fixed part to be attached to the teeth in the oral cavity; an image capturing apparatus that captures the drill marker and the fixed frame marker; a processing apparatus that calculates, on the basis of data from the image capturing apparatus, (i) a predicted implantation completion position of an implant corresponding to a current position of the drill blade and (ii) a current position of the implant corresponding to the current position of the drill blade; and a display device that displays the bones and the teeth into which the implant is to be embedded, displays a predetermined implant completion position of the implant in a first attribute, displays the predicted implantation completion position in a second attribute, and displays the current position in a third attribute.

[2-7]

A surgical procedure support system including: a fixed part attached to the teeth and having a fixed part marker; a fixed frame having a marker for fixed frame (fixed frame marker) and being mounted to the fixed part such that it extends out of the oral cavity from the fixed part; a drill frame having a drill marker and detachably attached to a dental drill having a drill blade; an image capturing apparatus that captures the drill marker and the fixed frame marker; a processing apparatus that stores in advance a positional relationship between the fixed part marker and the fixed frame marker; and a display device that displays the bones and the teeth into which an implant is to be embedded, wherein the processing apparatus calculates a positional relationship between the teeth and the fixed frame marker on the basis of (i) data obtained by capturing the fixed part in a state of being attached to the teeth, (ii) the positional relationship between the fixed part marker and the fixed frame marker, and (iii) a positional relationship between the drill marker and the fixed frame marker, and also calculates a predicted implantation completion position of an implant corresponding to a current position of the drill blade and a current position of the implant corresponding to a current position of the drill blade, and the display device that displays bone and the teeth into which the implant is to be embedded, displays a predetermined implantation completion position of the implant in a first attribute, displays the predicted implantation completion position in a second attribute, and displays the current position in a third attribute.

[2-8]

A registration apparatus including: a fixed part attached to the teeth and having a fixed part marker; a fixed frame having a fixed frame marker and being mounted to the fixed part such that it extends out of the oral cavity from the fixed part; an image capturing apparatus that captures the fixed frame marker; and a processing apparatus that stores in advance a positional relationship between the fixed part marker and the fixed frame marker, wherein the processing apparatus calculates a positional relationship between the teeth and the fixed frame marker on the basis of (i) data obtained by capturing the fixed part in a state of being attached to on the teeth and (ii) the positional relationship between the fixed part marker and the fixed frame marker.

[2-9]

The registration apparatus described in [2-8], wherein the fixed frame marker may have a plurality of angles with respect to the fixed part.

[Description of the reference numerals]

| | |
|---|---|
| 1 | Surgical procedure support system |
| 10 | Plate |
| 11 | CT marker |
| 12 | Detachable part |
| 15 | Stent |
| 16 | Recessed part |
| 17 | Plaster model |
| 20 | Reference frame |
| 25, 39 | Infrared markers |
| 30 | Manipulator |
| 40 | Infrared imaging apparatus |
| 50 | CT imaging apparatus |
| 60 | Display |
| 100 | Surgical procedure support apparatus |
| 110 | Storage part |
| 120 | Anonymization processing part |
| 130 | Input part |
| 140 | Position information acquisition part |
| 150 | Implant design information generation part |
| 160 | Display control part |
| 170 | Transmission part |
| 180 | Reception part |
| 190 | Position calculation part |
| 300 | Processing apparatus |
| 310 | Storage part |
| 320 | Segmentation processing part |
| 330 | Three-dimensional model generation part |
| 340 | Position acquisition part |
| 350 | Implant design information generation part |
| 360 | Three-dimensional measurement part |
| 370 | Coordinate calculation part |
| 380 | Registration part |
| 390 | Accuracy calculation part |
| 395 | Transmission part |
| 450 | Drill |
| 451 | Drill blade |
| 460 | Drill frame |
| 461 | Drill marker |
| 500 | Virtual implant |
| 550 | Pointing tool |
| 551 | Pointing part |
| 560 | Tool frame |
| 561 | Pointing maker |
| 650 | Driver tool |
| 651 | Driver |
| 660 | Driver frame |
| 661 | Driver marker |

**Claims**

1. A plate to be used by being fixed to a stent to be mounted on a row of teeth of a patient, the plate comprising:

a plurality of computed tomography (CT) markers;
a detachable part that is configured to connect a reference frame provided with a plurality of infrared markers whose relative positional relationship with the respective CT markers when the reference frame is connected to the plate is known, and
a recessed part that is configured to be used in measuring registration accuracy between (i) position coordinates of the plurality of CT markers in a three-dimensional reconstructed CT image including the plate as an object and (ii) position coordinates of the CT markers in real space in which the plate exists, the position coordinates being calculated on the basis of position coordinates of the plurality of infrared markers.

2. A processing apparatus, comprising:

a storage part that stores digital imaging and communications in medicine (DICOM) data relating to a CT image captured while a stent, to which the plate described in claim 1 is fixed, is attached to the teeth of a patient, the DICOM data being acquired via a network;
a three-dimensional model generation part that generates a three-dimensional reconstructed CT image on the basis of the DICOM data;
a position acquisition part that receives a designation of positions of the plurality of CT markers in the three-dimensional reconstructed image from a user of the processing apparatus;
a three-dimensional measurement part that measures the position coordinates of the plurality of infrared markers in a state where the stent, to which the plate connected with the reference frame is fixed, is mounted on a tooth-form plaster model of the patient;
a coordinate calculation part that calculates position coordinates of the plurality of CT markers provided on the plate on the basis of a relative positional relationship between the position coordinates of the plurality of infrared markers and the plurality of CT markers; and
a registration part that implements a registration of the three-dimensional reconstructed image and the tooth-form plaster model on the basis of the positions of the plurality of CT markers designated by the user and the position coordinates calculated by the coordinate calculation part.

3. The processing apparatus according to claim 2, wherein the reference frame rotates at a plurality of predetermined angles with respect to the plate fixed to the stent mounted on the tooth-form plaster model, and

the registration part generates a transformation matrix for transforming the positions of the plurality of infrared markers provided in the reference frame into the positions of the plurality of CT markers in the three-dimensional reconstructed image for each of the predetermined angles, on the basis of (i) known design information regarding the relative positional relationship between the plurality of infrared markers provided in the reference frame and the plurality of CT markers provided in the plate and (ii) the positions of the plurality of CT markers in the three-dimensional reconstructed image.

4. The processing apparatus according to claim 3, further comprising a transmission part that transmits the three-dimensional reconstructed image, the positions of the plurality of CT markers in the three-dimensional reconstructed image, and the transformation matrix for each of the predetermined angles to a transmission-source device, from which the DICOM data is transmitted, via the network.

5. The processing apparatus according to claim 4, wherein the three-dimensional measurement part further captures a drill frame having a plurality of drill markers whose relative positional relationship with the drill blade is known, the drill frame being detachably attached to a dental drill having a drill blade,

the registration part calculates a position of the drill blade in the three-dimensional reconstructed image on the basis of (i) position coordinates of the plurality of drill markers measured by the three-dimensional measurement part, (ii) a relative positional relationship between the plurality of drill markers and the drill blade, (iii) position coordinates of the plurality of infrared markers, and (iv) a relative positional relationship between the position coordinates of the plurality of infrared markers and the plurality of CT markers, and

the processing apparatus further includes an accuracy calculation part that calculates, as the registration accuracy, an error between a position of the drill blade and a position of the recessed part in the three-dimensional reconstructed image when the user inserts the drill blade into the recessed part; and

the transmission part further transmits the registration accuracy to the transmission-source device, from which the DICOM data is transmitted, via the network.

6. A surgical procedure support system for dental implant treatments including a surgical procedure support apparatus at a first facility where the dental implant treatments are performed and the processing apparatus according to claim 4 or 5 at a second facility different from the first facility and connected to the surgical procedure support apparatus via a network, wherein

the surgical procedure support apparatus comprises

a transmission part that transmits, to the processing apparatus via the network, DICOM data relating to the CT image captured in the state where the stent, to which the plate described in claim 1 is fixed, is mounted on the teeth of a patient,

a reception part that receives, from the processing apparatus via the network, the three-dimensional reconstructed image, the positions of the plurality of CT markers in the three-dimensional reconstructed image, and the transformation matrix for each of the predetermined angles, and

a display control part that causes a display to display the three-dimensional reconstructed image.

7. The surgical procedure support system according to claim 6, wherein in the surgical procedure support apparatus further comprises

a position information acquisition part that measures (i) position coordinates of the plurality of infrared markers included in the reference frame and (ii) position coordinates of the plurality of drill markers included in a drill reference frame having the drill markers whose relative positional relationship with a drill blade is known, the drill reference frame being detachably attached to a dental drill having the drill blade, and

a position calculation part that calculates a position of the drill blade in the three-dimensional reconstructed image on the basis of (i) the three-dimensional reconstructed image, (ii) the position coordinates of the plurality of infrared markers, (iii) the position coordinates of the plurality of drill markers, (iv) the relative positional relationship between the drill blade and the plurality of drill markers, and (v) the transformation matrix, wherein

the display control part causes the display part to display the drill blade superimposed on the three-dimensional reconstructed image.

8. The surgical procedure support system according to claim 7, wherein the display control part causes the display part to display (1) an image, which is extracted from the three-dimensional reconstructed image, including the bones into which the implant of the patient is to be embedded, (2) a predetermined implantation completion position of the implant, (3) a predicted implantation completion position of the implant calculated on the basis of the current position of the drill blade which is calculated by the position calculation part, and (4) a current position of the implant corresponding to the current position of the drill blade, in different modes.

9. The surgical procedure support system according to claim 8, further comprising a position information acquisition part that acquires position coordinates of a plurality of pointing markers in a pointing tool frame that has the pointing markers detachable to a pointing part for pointing out a point in real space, a relative positional relationship of the pointing markers with the pointing part is known, wherein

the position calculation part further calculates a position of the pointing part in the three-dimensional reconstructed image on the basis of (i) the three-dimensional reconstructed image, (ii) the position coordinates of the plurality of infrared markers, (iii) the position coordinates of the plurality of pointing markers, (iv) the relative positional relationship between the plurality of pointing markers and the pointing part, and (v) the transformation matrix, and the display control part causes the display part to display a virtual plane, which is parallel to a plane whose normal is the longitudinal direction of the implant at the predetermined implantation completion position of the implant in the three-dimensional reconstructed image and a distance from the position of the pointing part in the three-dimensional reconstructed image is a predetermined distance, by superimposing the virtual plane on an image including the bones into which the implant of the patient is to be implanted and the predetermined implantation completion position of the implant.

10. The surgical procedure support system according to claim 9, wherein

the position information acquisition part further acquires position coordinates of a plurality of driver markers included in a driver frame having the driver markers whose relative positional relation with a driver to be used to implant an orthodontic anchor screw is known, the driver markers being detachably attached to the driver, the position calculation part further calculates a position of the driver in the three-dimensional reconstructed image on the basis of (i) the three-dimensional reconstructed image, (ii) the position coordinates of the plurality of infrared markers, (iii) the position coordinates of the plurality of driver markers, (iv) the relative positional relationship between the plurality of driver markers and the driver, and (v) the transformation matrix, and

the display control part causes the display part to display a virtual image in which the driver is advanced along a long axis of the driver superimposed on an image including the bones in which the orthodontic anchor screws of the patient is to be embedded.

DENTAL CLINIC

PATIENT

10 20

11 25

30 39

CT IMAGING APPARATUS 50

INFRARED IMAGING APPARATUS 40

100 60

SURGICAL PROCEDURE SUPPORT APPARATUS

1

3D-MODEL TRANSFORMATION MATRIX, etc.

DICOM DATA, etc.

SECOND FACILITY (CLOUD SERVER)

THIRD FACILITY (DENTAL LABORATORY)

DICOM DATA, etc.

MANAGEMENT SERVER 200

PROCESSING APPARATUS 300

3D-MODEL TRANS-FORMATION MATRIX, etc.

FIG. 1A

| 50 | 40 |
|---|---|
| CT IMAGING APPARATUS | INFRARED IMAGING APPARATUS |

**100**

| | |
|---|---|
| STORAGE PART ~110 | POSITION INFORMATION ACQUISITION PART ~140 |
| ANONYMIZATION PROCESSING PART ~120 | IMPLANT DESIGN INFORMATION GENERATION PART ~150 |
| INPUT PART ~130 | DISPLAY CONTROL PART ~160 |
| TRANSMISSION PART ~170 | RECEPTION PART ~180 |
| POSITION CALCULATION PART ~190 | |

60

DISPLAY

**200**

| | |
|---|---|
| BACKUP DATABASE ~210 | LOG DATA DATABASE ~220 |
| SOFTWARE UPDATE PART ~230 | |

**300**

| | |
|---|---|
| STORAGE PART ~310 | POSITION ACQUISITION PART ~340 |
| SEGMENTATION PROCESSING PART ~320 | IMPLANT DESIGN INFORMATION GENERATION PART ~350 |
| 3D MODEL GENERATION PART ~330 | 3D MEASUREMENT PART ~360 |
| COORDINATE CALCULATION PART ~370 | REGISTRATION PART ~380 |
| ACCURACY CALCULATION PART ~390 | TRANSMISSION PART ~395 |

40

INFRARED IMAGING APPARATUS

FIG. 1B

FIG. 2

(a)

(b)

(c)

(d)

FIG. 3

FIG. 4

FIG. 5

| 0° | X | Y | Z |
|---|---|---|---|
| 11(1) | −100 | 9 | 6 |
| 11(2) | −90 | 10 | 4 |
| 11(3) | −70 | 20 | 5 |
| 11(4) | −70 | 40 | 4 |
| 11(5) | −80 | 50 | 5 |
| 11(6) | −90 | 50 | 6 |
| 11(7) | −100 | 55 | 5 |

| 60° | X | Y | Z |
|---|---|---|---|
| 11(1) | −65 | 9 | −75 |
| 11(2) | −60 | 10 | −70 |
| 11(3) | −50 | 20 | −50 |
| 11(4) | −50 | 40 | −55 |
| 11(5) | −55 | 50 | −60 |
| 11(6) | −60 | 50 | −65 |
| 11(7) | −65 | 55 | −75 |

| 30° | X | Y | Z |
|---|---|---|---|
| 11(1) | −90 | 9 | −45 |
| 11(2) | −80 | 10 | −40 |
| 11(3) | −70 | 20 | −30 |
| 11(4) | −70 | 40 | −30 |
| 11(5) | −75 | 50 | −30 |
| 11(6) | −85 | 50 | −35 |
| 11(7) | −90 | 55 | −45 |

| 90° | X | Y | Z |
|---|---|---|---|
| 11(1) | −20 | 9 | −90 |
| 11(2) | −15 | 10 | −80 |
| 11(3) | −19 | 20 | −65 |
| 11(4) | −15 | 40 | −65 |
| 11(5) | −19 | 50 | −70 |
| 11(6) | −20 | 50 | −80 |
| 11(7) | −19 | 55 | −90 |

EXAMPLE OF RELATIVE POSITIONS OF RESPECTIVE REFERENCE MARKS 11(1) TO 11(7) IN XYZ SPACE, WITH CENTER O AS ITS ORIGIN, FOR EVERY ANGLES (0°, 30°, 60°, 90°) (CAD data)

FIG. 6

EP 3 925 568 A1

START

CREATE DENTAL IMPRESSION, PLASTER MODEL, AND STENT — S1

MOUNT REFERENCE PLATE 10 ON STENT — S2

FIX REFERENCE PLATE 10 TO THE TEETH OF PATIENT, AND TAKE CT IMAGES — S3

TRANSMIT DICOM DATA AND THE LIKE TO MANAGEMENT SERVER 200 — S4

} SURGICAL PROCEDURE SUPPORT APPARATUS 100

STORE DICOM DATA AND TRANSMIT THE SAME TO PROCESSING APPARATUS 300 — S5

} MANAGEMENT SERVER 200

CREATE DENTAL IMPRESSION, PLASTER MODEL, AND STENT — S6

REGISTRATION PROCESSING, GENERATION OF TRANSFORMATION MATRIX — S7

CREATE DESIGN INFORMATION (OPTIONAL) — S8

TRANSMIT PROCESSED DICOM DATA AND THE LIKE TO MANAGEMENT SERVER 200 — S9

} PROCESSING APPARATUS 300

STORE PROCESSED DICOM DATA AND THE LIKE, AND TRANSMIT THE SAME TO SURGICAL PROCEDURE SUPPORT APPARATUS 100 — S10

} MANAGEMENT SERVER 200

CREATE DESIGN INFORMATION — S11

IMPLANT TREATMENTS — S12

} SURGICAL PROCEDURE SUPPORT APPARATUS 100

END

FIG. 7

(a)

17    15

(b)

10

15

FIG. 8

(a)

CT IMAGE OF PATIENT (SLICE)

FIG. 9

(b)

THREE-DIMENSIONAL MODEL OF CT
IMAGES OF PATIENT (3D)

FIG. 10

IMAGE OF 3D MODEL INCLUDING DESIGN
POSITION OF IMPLANT

FIG. 11

(A) DESIGNING

(B) (C) APPROACHING OF DRILL

(D) OVERLAP WITH DESIGN POSITION

(E) START DRILLING

(F) REACHED GOAL AND COMPLETED

(G) OVERDRILLING

FIG. 12

FIG. 13

(a)

(b)

FIG. 14

## INTERNATIONAL SEARCH REPORT

| International application No. |
| --- |
| PCT/JP2020/002160 |

**A. CLASSIFICATION OF SUBJECT MATTER**
A61C 8/00(2006.01)i; A61B 34/10(2016.01)i; A61C 19/04(2006.01)i
FI: A61C8/00 Z; A61B34/10; A61C8/00 A; A61C19/04 Z
According to International Patent Classification (IPC) or to both national classification and IPC

**B. FIELDS SEARCHED**

Minimum documentation searched (classification system followed by classification symbols)
A61C8/00; A61B34/10-34/20; A61C19/04

Documentation searched other than minimum documentation to the extent that such documents are included in the fields searched

| | |
| --- | --- |
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| Y | WO 2018/088146 A1 (KYUSHU UNIVERSITY) 17.05.2018 (2018-05-17) paragraphs [0025]-[0053], fig. 1-12 | 1 |
| Y | US 2017/0196521 A1 (HUANG, Jerry T.) 13.07.2017 (2017-07-13) paragraphs [0002], [0030]-[0042], fig. 3-15 | 1 |
| A | WO 2018/143497 A1 (KIM, Do-Hyun) 09.08.2018 (2018-08-09) paragraphs [0060]-[0098], fig. 1-6 | 2-10 |
| A | WO 2018/066765 A1 (MEGAGEN IMPLANT CO., LTD.) 12.04.2018 (2018-04-12) paragraphs [0060]-[0078], [0098]-[0111], fig. 1-7 | 2-10 |
| A | US 2013/0122463 A1 (CSILLAG, Raphael Yltz) 16.05.2013 (2013-05-16) paragraphs [0081]-[0115], fig. 5-6 | 2-10 |

☒ Further documents are listed in the continuation of Box C. ☒ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 01 April 2020 (01.04.2020) | 21 April 2020 (21.04.2020) |

| Name and mailing address of the ISA/ | Authorized officer |
| --- | --- |
| Japan Patent Office | |
| 3-4-3, Kasumigaseki, Chiyoda-ku, | |
| Tokyo 100-8915, Japan | Telephone No. |

Form PCT/ISA/210 (second sheet) (January 2015)

INTERNATIONAL SEARCH REPORT

| International application No. |
| --- |
| PCT/JP2020/002160 |

C (Continuation).   DOCUMENTS CONSIDERED TO BE RELEVANT

| Category* | Citation of document, with indication, where appropriate, of the relevant passages | Relevant to claim No. |
| --- | --- | --- |
| A | US 2015/0140505 A1 (NATIONAL CHUNG CHENG UNIVERSITY) 21.05.2015 (2015-05-21) fig. 3 | 1 |

Form PCT/ISA/210 (continuation of second sheet) (January 2015)

Form PCT/ISA/210 (patent family annex) (January 2015)

**INTERNATIONAL SEARCH REPORT**
Information on patent family members

International application No.

PCT/JP2020/002160

| Patent Documents referred in the Report | Publication Date | Patent Family | Publication Date |
|---|---|---|---|
| WO 2018/088146 A1 | 17 May 2018 | (Family: none) | |
| US 2017/0196521 A1 | 13 Jul. 2017 | US 9554869 B1 | |
| WO 2018/143497 A1 | 09 Aug. 2018 | JP 2020-506033 A paragraphs [0060]-[0098], fig. 1-6 US 2019/0380811 A1 | |
| WO 2018/066765 A1 | 12 Apr. 2018 | JP 2019-531173 A paragraphs [0043]-[0056], [0076]-[0089], fig. 1-7 EP 3524139 A1 KR 10-2018-0038319 A | |
| US 2013/0122463 A1 | 16 May 2013 | WO 2013/074784 A1 | |
| US 2015/0140505 A1 | 21 May 2015 | US 2014/0147807 A1 | |

**EP 3 925 568 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2018088146 A
- US 20180279975